# EUROPEAN PATENT APPLICATION

(11) **EP 4 272 800 A1**
(43) Date of publication of application: **08.11.2023**
(21) Application number: 22315100.2
(22) Date of filing: 06.05.2022
(51) Int. Cl.: A61M 25/01, A61B 17/34, A61B 34/00, A61B 34/20, A61M 25/06, A61M 29/00, A61B 6/02, A61B 8/12, A61B 8/06, A61B 8/08, A61B 8/00, A61B 90/00, A61B 90/50, A61B 34/30

(54) **A BIOMEDICAL DEVICE, A SYSTEM COMPRISING A BIOMEDICAL DEVICE, A METHOD FOR PERCUTANEOUS CATHETERIZATION OF A BODY DUCT, AND A METHOD FOR ALIGNING AN EXTERNAL IMAGING MEANS**

(71) Applicant: Caranx Medical SAS, 75013 Paris (FR)
(72) Inventor: SMITS, Jonas Victor Hamen, 3360 Bierbeek (BE); SEJOR, Eric, 06000 Nice (FR); CERRUTI, Giulio, 06700 Saint-Laurent-du-Var (FR); BERTHET-RAYNE, Pierre, 06800 Cagnes-sur-Mer (FR)
(74) Representative: Hepp Wenger Ryffel AG

(57) **Abstract**

The invention relates to a biomedical device (101) for percutaneous catheterization of a body duct (13), in particular arterial catheterization, the device (101) comprising an introducer assembly (1) which has a dilator (2) for widening of an insertion site (131) of a patient (14) to access the body duct (13), a sheath (3) for introduction into the body duct (13), and an inner guiding unit (4) for guiding the dilator (2) and/or the sheath (3) into the body duct (13), the dilator (2) having a tapered outer section (21) and at least one distal opening (22), and being arranged at least partially concentrically around the guiding unit (4) and the sheath (3) having at least one distal opening (32) and being arranged at least partially concentrically around the dilator (2), such that
the guiding unit (4), the dilator (2), and the sheath (3) are movable relative to each other in a longitudinal direction (L) of the introducer assembly (1),characterized in that the dilator (2) may be guided along the guiding unit (4) without removal of a puncturing element (41) for puncturing the body duct (13) at a proximal end (5) of the introducer assembly (1) after insertion of the puncturing element (41) into the body duct (13).

## Description

The invention relates to a biomedical device, a system comprising a biomedical device, a method for percutaneous catheterization a body duct, and a method for aligning external imaging means relative to a body duct.

Introduction of a catheter in a percutaneous procedure for the purpose of catheterization requires puncturing vascular body ducts, in particular arterial body ducts. This placement of a catheter in an arterial and/or venous system is often performed in the prior art by using the Seldinger technique.

The Seldinger technique comprises placing a cannula within the body duct and introducing a guidewire being circumferentially surrounded by the cannula through the cannula into the body duct. Subsequently, the cannula is retracted from a proximal end of the guidewire, such that a dilator and finally a catheter sheath can be advanced over the guidewire into the body duct.

US 5,7493,71 A discloses a device for automated guidewire placement using the Seldinger technique with a hollow cannula with a cannula tip with gripping means for releasably engaging a guidewire.

N. Zevallos et al., "Toward Robotically Automated Femoral Vascular Access," 2021 International Symposium on Medical Robotics (ISMR) discloses a robot-assisted arterial catheterization through the Seldinger technique.

A particularly time-consuming and complex step, in particular of a non-manual automated implementation of the Seldinger technique, is the removal of the cannula by fully retracting the coaxial cannula over the guidewire.

In general, the prior art lacks a simple, reliable, and timesaving method to enable percutaneous catheterization, in particular automated percutaneous catheterization, of a body duct.

It is the object of the present invention to overcome these and other disadvantages in the prior art. In particular, the invention shall provide a device and system that reduces and/or optimizes the number of steps required for surgical catheterization, particularly catheterization using the Seldinger technique, and provides a standardized technique for atraumatic catheterization.

Another object of the invention is to provide a biomedical device and system with reduced complexity.

The object is solved by a biomedical device, a system comprising a biomedical device, a method for percutaneous catheterization, and a method for aligning an external imaging means according to the independent claims. Further embodiments are defined in the dependent claims.

A biomedical device according to the invention is used for percutaneous catheterization of a body duct, in particular arterial catheterization. The device comprises an introducer assembly which has a dilator for widening of an insertion site of a patient to access the body duct. The device further comprises a sheath for introduction into the body duct and an inner guiding unit for guiding the dilator and/or the sheath into the body duct. The dilator has a tapered outer section and at least one distal opening and is or can be arranged at least partially concentrically around the guiding unit. The sheath has at, least one distal opening and is arranged at least partially concentrically around the dilator.

Once the puncturing element is inserted into the body duct, the dilator may be guided along the guiding unit without complete withdrawal of the puncturing element from a proximal end of the introducer assembly.

The proximal end refers to the end of the introducer assembly that is locatable outside of the patient proximally relative to an insertable distal end of the introducer as perceived by a surgeon.

The introducer assembly can be a consumable introducer assembly having pre-aligned movable parts for usage with a robotic actuation.

The guiding unit can comprise or consist of a guidewire, the puncturing element, and/or an insertion guide.

The puncturing element can be a cannula, optionally a commercially available cannula with a hollow cylindrical cannula shape and a sharpened distal end which is sharpened with an oblique grind.

The tapered outer sections of the dilator and sheath can be configured for atraumatic introduction into the body duct over the guiding unit.

The guiding unit can comprise an insertion guide for inserting the puncturing element or the guidewire. The insertion guide has a distal section and a proximal section. The proximal section has a proximal lumen for at least partially receiving the puncturing element or the guidewire. The distal section has a distal lumen for receiving the puncturing element or the guidewire. The distal lumen and the proximal lumen are connected to each other. In this embodiment, the distal section has a lateral opening at a proximal end through which the puncturing element can be reversibly inserted into the distal lumen and a distal opening from which the puncturing element can be inserted into the body duct.

The distal section of the insertion guide can be insertable into the body duct with the puncturing element projecting from its distal opening.

The puncturing element does not need to be fully retracted from the proximal end of the introducer assembly but instead can be retracted from the lateral opening which is arranged at an intermediate position. Thereby, the catheterization is made easier. In addition, the insertion guide can be inserted into the body duct simultaneously with the puncturing element which further simplifies the catheterization procedure.

The openings of the device, in particular the distal opening, the lateral opening, the at least one distal opening of the dilator and/or the sheath can comprise a hemostatic valve for reducing leakage and/or blood backflow.

The distal lumen of the insertion guide can extend in a straight line between the lateral opening and the distal opening of the distal section.

Alternatively, the distal section of the insertion guide can be elastically deformable, such that the distal lumen can assume the shape of a straight line between the lateral and distal opening.

The distal lumen of the insertion guide extending in a straight line allows for easy insertion and removal of a puncturing element which may have a straight shape and which may not be readily bendable.

The length of the distal lumen in a longitudinal direction of the insertion guide is preferably sufficient to reliably guide the puncturing element into the body duct and short enough to allow rapid insertion and removal of the puncturing element which is typically in the form of a cannula. The length of the distal lumen can range from 1 cm to 20 cm, preferably 7 cm to 20 cm.

A longitudinal axis of the proximal section of the insertion guide can be arranged or arrangeable at an angle of 1° to 125°, preferably an angle of 10° to 75°, particularly 20° to 50°, relative to a longitudinal axis of the distal section of the insertion guide.

Such an angle allows for reliable insertion of the puncturing element and at the same time allows a guidewire to be insertable through the proximal and distal lumen of the insertion guide. The guidewire is sufficiently flexible to conform to the angle between the distal and proximal section of the insertion guide.

The dilator can be arranged or arrangeable coaxially around the insertion guide and configured to be advanced relative to the insertion guide in the longitudinal direction of the insertion guide.

The dilator can also be arranged or arrangeable coaxially around a guidewire and configured to be advanced relative to the guidewire in a longitudinal direction of the guidewire. A mantle section of the proximal and distal section can then have a release opening extending in a longitudinal direction. The release opening can be permanently open or openable. The release opening is configured for radially releasing the guidewire which is at least partially arranged or arrangeable in the proximal and distal lumen.

A dilator arranged coaxially around the insertion guide and advanceable relative to the insertion guide in the longitudinal direction allows for easy introduction of the dilator into the body duct over the proximal and distal section of the insertion guide.

The distal and proximal sections of the insertion guide can have a constant cross-sectional dimension along their length, so that the dilator can be advanceable over the complete insertion guide.

The insertion guide can extend at least partially, in particular completely, through an inner lumen of the dilator.

A dilator arranged coaxially around the guidewire and advanceable relative to the guidewire in the longitudinal direction allows for using existing commercially available components for the dilator, the sheath, and the guidewire which are used for performing the Seldinger technique. The guidewire can be removed radially from the introducer guide, such that the introducer guide does not have to be removed through the proximal end of the device. Thus, the guidewire can stay in place and the introducer guide can be removed from the patient's site before the dilator is advanced over the guidewire to insert the introducer assembly.

The dilator and/or the sheath can comprise a hydrophobic coating which allows for an atraumatic insertion into the body duct.

The dilator and/or the sheath can have a size between 1 Fr and 34 Fr, in particular 3 Fr to 16 Fr, wherein 1 Fr is equal to 0.33 mm.

The dilator and/or the sheath can comprise or consist of thermoplastics such as silicones, polyethylene, polypropylene, polyetheretherketone, fluoropolymers, polyvinyl chloride and/or polyurethane.

The device can comprise multiple dilators and/or sheaths.

A guidewire of the guiding unit can be pre-aligned within a proximal lumen of a proximal section of an insertion guide of the guiding unit.

The guiding unit and in particular the insertion guide, the guidewire, and/or the puncturing element, can comprise or consist of a metal, in preferably stainless steel or a shape memory alloy, particularly nitinol.

The puncturing element can have a hollow cylindrical cannula-shape.

The puncturing element can comprise at least one retaining structure. The retaining structure can have a recess on an outer surface and open radially outwardly for receiving a guidewire extending at least partially along a longitudinal direction of the puncturing element.

The retaining structure may also have a lumen defined by a wall which is at least partially open or openable for radially releasing a guidewire which is at least partially arranged or arrangeable in the lumen.

The retaining structure can alternatively have an attachment mechanism for attaching a guidewire in parallel to the puncturing element.

The retaining structure can comprise a retaining state for retaining the guidewire at the puncturing element and a releasing state for radially releasing the guidewire from the lumen or recess of the puncturing element.

The retaining structure can be convertible, optionally reversibly convertible, from the retaining state to the releasing state, e.g. by a rotational movement or a translational movement of the retaining structure and/or the guidewire relative to each other or to the introducer assembly.

The retaining structure can comprise a spring-loaded tip of the puncturing element. The spring-loaded tip can be converted from the retaining state to the releasing state. The spring-loaded tip is convertible to the retaining state when compressed and to the releasing state when decompressed. Thereby, the spring-loaded tip can retain the guidewire during introduction through skin and tissue into the body duct while being compressed and can release the guidewire within the body duct based on a reduction of the compressive force within the body duct.

The retaining structure can comprise a mechanical coupling element to frictionally engage the puncturing element, an, optionally bioresorbable, glue, or a lumen within the puncturing element for applying a local suction to retain the guidewire. The guidewire may have a lumen for applying the local suction for retaining the guidewire.

The retaining structure can be convertible by applying local vibration to overcome frictional engagement of the mechanical coupling element. The retaining structure can also be convertible by eliminating a connection provided by the, preferably bioresorbable, glue. The retaining structure may be convertible from a retaining state, in which suction is applied to the lumen of the puncturing element or guidewire, to a releasing state by ceasing local suction on the lumen.

Thereby, the retaining structure may be converted to the releasing state for releasing the guidewire once the guidewire has been inserted into the body duct.

The open or openable lumen can comprise at least one, in particular two, mantle sections. A first and a second mantle section can be movable relative to each other. The first and second mantle sections can form a C-shape, in particular form a C-shape with different diameters, such that one of the mantle sections can be positioned to be at least partially enclosed by the other of the mantle sections.

The recess or lumen can be arranged partially along the longitudinal direction and partially in a circumferential direction of the puncturing element, in particular along a helical or curved path. Such a helical or curved path can impede movement of the guidewire in the longitudinal direction of the puncturing'element relative to the puncturing element due to increased friction.

To increase a static and/or a sliding friction, the recess or the lumen of the puncturing element can be configured to frictionally engage the guidewire.

The puncturing element can be relatively long and can have a length of 6 cm to 35 cm, preferably 8 cm to 30 cm, more preferably 12 cm to 25 cm. The proximal end of the puncturing element can have a'connection to a distal end of the guidewire, such that the puncturing element and the guidewire are essentially flush with each other.

A relatively long puncturing element allows to introduce the dilator into the body duct coaxially around the puncturing element directly, in particular without requiring a guidewire.

A puncturing element connected to the distal end of the guidewire has the advantage, that the longitudinal length of the puncturing element can be shorter than 6 cm, less than 2 cm, preferably less than 1 cm. Due to a shorter puncturing element which is rather stiff, the dilator can be advanced more reliably along the more flexible guidewire section when it is introduced into the body duct.

The puncturing element and guidewire can each have different elastic properties. The guidewire can e.g. have a lower bending stiffness than the puncturing element such that a curvature of the guidewire can be adapted to the body duct and/or the introducer assembly more easily.

The guidewire connected to the puncturing element can still function as a push rod which can be moved in the longitudinal direction relative to the introducer assembly to introduce the puncturing element. The dilator can be introducible into the body duct coaxially around the puncturing element which is connected to the guidewire.

A puncturing element connected to the distal tip of the end of the guidewire allows to avoid the traditional steps of the Seldinger technique, i.e. separately introducing the guidewire after introduction of the puncturing element and retracting the puncturing element once the guidewire was introduced into the body duct.

The puncturing element can be connected or connectable to the dilator and comprise an actuating element, in particular a spring, which is convertible between a retracted state and an extended state. By conversion of the actuating element, a puncturing section of the puncturing element for contacting and puncturing tissue is retractable behind a non-puncturing surface, or a non-puncturing surface can be advanced for shielding the puncturing section.

The puncturing element can then be used for piercing tissue and otherwise be rendered atraumatic by the non-puncturing surface. In addition, the dilator can be directly introduced by using the puncturing element which has a non-puncturing surface shielding the puncturing surface, without necessarily requiring a guidewire and/or an insertion guide.

The non-puncturing surface can be a located on a distal end of the dilator or on a separate element of the introducer assembly.

The non-puncturing surface and/or the actuating element may be arranged concentrically within the lumen of the puncturing element. The puncturing element may in particular be a Veress cannula.

The puncturing element can comprise an anchoring member arranged at least partially circumferentially on the puncturing element and preferably forming a longitudinal section of the puncturing element. The anchoring member comprises a collapsed configuration and an expanded configuration. The collapsed configuration has a radial dimension which is smaller than a radial dimension in the expanded configuration.

In the expanded configuration the anchoring member can prevent advancement of the puncturing element into the patient, in particular into the body duct, beyond a predetermined longitudinal distance of the puncturing element.

The anchoring member in the expanded configuration can additionally or alternatively prevent retraction of the puncturing element from the body duct beyond a predetermined longitudinal distance of the puncturing element, in particular by contacting an inner wall of the body duct. This can provide secure anchoring of the puncturing element within the body duct and prevent accidental removal of the puncturing element from the body duct.

Such an anchoring member prevents potential injuries caused by inserting the puncturing element too deeply into the skin of a patient or the body duct of the patient. In addition, such a puncturing element can be used for direct insertion of the dilator over the puncturing element, so that a guidewire and/or an insertion guide are not necessarily required.

The predetermined longitudinal distance may be shorter than the width of the body duct or greater than the distance from a puncture position of the puncturing element on a patient's skin to the body duct. The anchoring member can contact the skin of the patient or an outer wall of the body duct in the expanded configuration.

The dilator can comprise an anchor actuator, such that upon longitudinal movement of the dilator relative to the puncturing element, the anchor actuator engages the anchoring member in the expanded configuration to convert the anchoring member from the expanded configuration to the collapsed configuration.

The dilator can comprise longitudinal slots arranged neighboring the distal opening of the dilator. The longitudinal slots are configured to be advanced past the anchoring member in the longitudinal direction of the puncturing element.

The anchor actuator may comprise a radially extending protrusion arranged at least partially circumferentially on the puncturing element and spaced by a predetermined longitudinal distance from the anchoring member, such that the protrusion can be engaged by the dilator with slots being advanced over the anchoring member.

The anchoring member can be biased toward the expanded configuration, in particular biased due to a restoring element, in particular by a spring element, which exerts a restoring force on the anchoring member. Thereby, the anchoring member assumes the expanded configuration as a default state when not being retained or engaged by the anchor actuator of the dilator. The sheath, the dilator, and/or at least a part of the guiding unit, in particular the puncturing element, can be connected to at least one mechanical coupling. A relative longitudinal movement of one of these components (the sheath, the dilator, and/or at least the part of the guiding unit) beyond a predetermined point causes the coupling to move another of these components in the longitudinal direction.

The coupling allows the movement of the components of the introducer assembly, in particular the dilator, the sheath, and at least the part of the guiding unit, in particular the puncturing element, to be coupled or decoupled with each other.

The coupling may be configured to couple the components only mono-directionally, in particular in the distal direction or proximal direction of the introducer assembly, or couple the components bidirectionally, in the proximal direction and the distal direction of the introducer assembly.

The coupling can comprise at least one, in particular two, coupling elements.

The coupling can comprise a first coupling element located on one of the sheath, the dilator, or the part of the guiding unit and a second coupling element located on the other of the sheath, the dilator; or the part of the guiding unit. The coupling elements can be formed to positively engage each other at the predetermined point.

The coupling can comprise at least one recess and/or at least one protrusion extending in the radial direction.

The recess and/or the protrusion can be arranged at least partially in a circumferential direction of the dilator, sheath or at least the part of the guiding unit, in particular the puncturing element.

A system according to the invention comprises a biomedical device, preferably a previously described biomedical device, for percutaneous catheterization of a body duct, in particular arterial catheterization. The biomedical device comprises an introducer assembly which has a dilator for widening of an insertion site of the patient to access the body duct, a sheath for introduction into the body duct, and an inner guiding unit for guiding the dilator and/or the sheath.

The dilator has a tapered outer section, a distal opening, and is arranged at least partially concentrically around the guiding unit. The sheath has a distal opening and is arranged at least partially concentrically around the dilator.

The guiding unit, the dilator, and the sheath are movable relative to each other in a longitudinal direction of the introducer assembly. The system comprises at least one actuation unit which is configured to actuate the dilator, the sheath, and/or the guiding unit, in particular a puncturing element, a guidewire, and/or an insertion guide, of the device. The actuating movement is a movement in a longitudinal direction of the introducer assembly.

The system can comprise at least one actuation unit, preferably exactly one actuation unit, which comprises a clutch, in particular a clutch comprising at least two rollers which have an adjustable interdistance. The actuation of the actuation unit can be switched between configurations for actuating the dilator, the sheath, and/or the guiding unit, in particular the puncturing element, the guidewire, and/or an insertion guide.

The rollers can be configured for frictionally engaging the dilator, the sheath, and/or the guiding unit.

According to another aspect of the invention, a system comprises a biomedical device, preferably a previously described biomedical device, for percutaneous catheterization of a body duct, in particular arterial catheterization. The device comprises an introducer assembly for puncturing an application site at a patient's skin. The introducer assembly comprises a positionable and actuatable cannula for puncturing the skin at a desired place and with a desired direction.

The device comprises an external imaging means, in particular an ultrasound probe, e.g. a Doppler ultra-sound probe for measuring a blood flow of the body duct, or a X-ray imaging probe. The imaging means are configured for imaging a body duct of a patient, in particular an arterial body duct, in two dimension or three dimensions, optionally in a time periodic measurement. The external imaging means and the introducer assembly are connected to a common support of the system.

The external imaging means and the introducer assembly, in particular the guiding unit, can be configured to be fixedly pre-oriented with respect to each other. A longitudinal axis of the field of view of the external imaging means and the introducer assembly may be arranged pre-oriented at an angle of 1° to 90°, in particular 20° to 60°, in particular preferably 30° to 45°, to each other.

The angle, a translational movement, and/or a rotational movement, between the imaging means and the introducer assembly may be adjustable by an alignment element, in particular at least one clamp or screw.

Additionally, the imaging device may be configured to image at least one other anatomical feature and generate data based on this other anatomical feature for the purpose of path planning. The anatomical feature may also be provided by or a preoperative scan for this purpose. The anatomical feature may be the position and orientation of nerves, ligaments, bones, fat, muscle, organ tissue, bifurcations, arterial walls, and calcifications.

The system can comprise an external guidance means. The external guidance means can comprise a stereo camera or a RGBD camera, a LIDAR system, and/or an electromagnetic sensor. Based on these guidance means a distance of the patient, in particular the body duct, and/or a surrounding of the patient to the imaging means and/or the introducer assembly, in particular the guiding unit, can be determined or reconstructed.

The system may have a landmark which comprises or consists of one or a plurality of visual recognition elements, in particular fiducials. The visual recognition elements can have a particular shape, in particular a line shape, a polygon shape or circular shape.

The plurality of visual recognition elements can have a predetermined distance to each other, such that the guidance means can be calibrated by measuring the distance of the visual recognition elements from the guidance means.

The visual recognition elements, in particular a patch, may be applied to or arranged on a drape or attached to the patient, in particular close to an insertion site of the patient to access the body duct.

The guidance means can be immovably connected to the external imaging means, immovably connected to the patient or a stationary surface, and/or attached to a movable surface which is independently movable from the imaging means.

The device of the system can comprise an introducer positioner for positioning the introducer assembly relative to the body duct and/or the common support. The introducer positioner can be configured to position the introducer assembly in two translational degrees of freedom and at least one rotational degree of freedom, or three translational degrees of freedom and at least one rotational degree of freedom, in particular two or three rotational degrees of freedom.

The common support of the system can be a hand-held support for manual alignment of the device, in particular the imaging means, relative to the patient.

It can also be a spatially fixed support which comprises a prepositioning means, in particular an adhesive, a strap, or a mechanically fixed or fixable carrier.

Alternatively, it can also be a spatially movable support which comprises a device positioner for positioning the device relative to the body duct independently in two or three translational degrees of freedom and at least one rotational degree of freedom, in particular one, two, or three rotational degrees of freedom.

The device positioner and/or the introducer positioner allows to align the introducer assembly, in particular the guiding unit, in translational and rotational degrees of freedom at the insertion site, in particular to a predetermined orientation relative to the body duct and/or the at least one visual recognition element.

The device positioner may be configured, preferably by the processing unit, to align the external imaging means with respect to the visual recognition element, in particular a visual recognition element placed on or in a proximity of the body duct.

The external imaging means being placed on or in the proximity of the body duct may be configured, preferably by the processing unit, to locally image the body duct, such that the processing unit can determine an insertion path based on the imaged body duct.

The introducer assembly may be positionable, preferably by the introducer positioner, based on the determined insertion path with respect to the body duct, such that the previously described insertion can be performed.

The predetermined orientation of the longitudinal direction of the introducer assembly, in particular the guiding unit, relative to the body duct may be at an angle of 1° to 80°, in particular 5° to 60°, preferably 10° to 55° to an axial direction of the body duct.

The introducer positioner and/or the device positioner can comprise at least, one driving element, in particular an electric motor, a hydraulic actuator, a pneumatic actuator, and/or a piezo motor.

The system can comprise a force sensor which is configured for converting an external force, in particular an external torque, exerted on at least a subsection of the system, in particular a distal tip of the guiding unit, preferably a distal end of the puncturing element of the guiding unit, to an electrical signal indicative of the external force.

Such a force sensor can verify the position and/or orientation of the subsection of the system, in particular the puncturing element, relative to a patient or the body duct.

The system can comprise a processing unit for processing data of the measured image of the body duct, in particular a plurality of images, of the external imaging means.

Alternatively or additionally, the processing unit can process the determined or reconstructed distance, in particular a plurality of distances, of the guidance means.

It can also process the electrical signal, in particular a plurality of electrical signals, of the force sensor. The data can be processed by the processing unit for calculating at least one electrical signal for operating the introducer positioner and/or the device positioner.

Information derived from the electrical signal processed by the processing unit can alternatively or additionally be displayed on a display unit of the system.

The data of the imaging device relative to the at least one other anatomical feature may be processed by the processing unit for determining at least one signal for operating the introducer positioner and/or the device positioner.

The processing unit can be configured for operating the introducer positioner in a partially or fully automated manner, in particular requiring only partial or no manual operating input of a user.

The system can comprise a memory unit connected or connectable to the processing unit for saving data of the measured image of the body duct, in particular a plurality of images, of the external imaging means. Additionally or alternatively, the memory unit can be configured for saving data of the determined or reconstructed distance, in particular a plurality of distances, of the guidance means.

It can also be configured for saving data of the electrical signal, in particular a plurality of electrical signals, of the force sensor.

The data can be saved at an initial point in time. The processing unit can further be configured for retrieving, the saved data at a later point in time from the memory unit for operating or supporting the operation of the introducer positioner and/or the device positioner.

The memory unit can be configured for saving the data of a preoperative scan or reconstruction, in particular a CT, MRI, or ultrasound scan or reconstruction.

The processing unit may be configured to register the preoperative or an intraoperative scan or reconstruction, in particular a CT, MRI, or ultrasound scan or reconstruction, optionally stored on the memory unit, to the image generated by the external imaging means.

The processing unit can be configured for using the saved and/or processed data to reconstruct an occupancy map which detects free spaces without obstacles.

The processing unit may be configured for determining the position of the insertion site and/or orientation of the introducer assembly, in particular the guiding unit, to avoid or minimize interference with calcifications within the body duct. The calcifications may be detected by the imaging means or the preoperative scan, particularly a CT scan.

The processing unit can be configured for processing the saved and/or processed data using an artificial intelligence, in particular by unsupervised, supervised, or reinforcement machine learning.

According to another aspect of the invention the object is further solved by a method for percutaneous catheterization of a body duct, preferably using a previously described biomedical device with an introducer assembly. The method comprises the following steps:
- Inserting an inner guiding unit through a skin of a patient into the body duct using a puncturing element,
- Optionally inserting a guidewire of the inner guiding unit through the insertion guide into the body duct,
- Guiding a dilator along the guiding unit without full removal of the puncturing element at a proximal end of the introducer assembly,
- Advancing a sheath over the dilator into the body duct,
- Optionally retracting the dilator, the inner guiding unit, in particular the insertion guide, and/or the guidewire.

The method can comprise the step of releasing the guidewire from a retaining structure of the puncturing element of the introducer assembly.

The method can comprise the step of measuring the blood flow through the guiding unit, in particular the insertion guide, which is introduced into the body duct.

The method can comprise the step of exchanging the guidewire used for the insertion for a more flexible guidewire for navigation within the body duct.

The object is further solved by a method for aligning an external imaging means relative to a body duct, preferably using a system as previously described. The method comprises the steps of:
- imaging a plane of the body duct using the external imaging means,
- aligning the external imaging means in translational degrees of freedom and/or rotational degrees of freedom of the imaging means, preferably using the device positioner, such that the body duct is centred in a field of view of the imaging means and positioned along an orthogonal direction of the imaging plane or in a range of 30°, in particular 15°, relative to the orthogonal direction,
- aligning the external imaging means in the translational and/or rotational degrees of freedom, preferably using the device positioner, such that the body duct remains centred in the field of view and the imaging plane is positioned at a parallel direction of a longitudinal axis of the body duct.

A longitudinal direction of the imaging plane can in addition also be positioned orthogonal to the longitudinal axis of the body duct.

The method can also comprise the step of registering data of a preoperative scan based on one other anatomical feature, in particular calcifications, to the body duct imaged by the external imaging means.

The method can further comprise the step of aligning the external imaging means in the translational and/or rotational degrees of freedom, preferably using the device positioner, such that this registered anatomical feature is avoided by the insertion path of the insertion of an introducer assembly.

The invention is now described with reference to certain embodiments and figures which show:
Figures 1A and 1B: longitudinal cross-sectional views of a first and second embodiment of a biomedical device,
Figure 2A: a longitudinal cross-sectional view of a third embodiment of a biomedical device comprising an insertion guide,
Figure 2B: a schematic arrangement of an actuation unit of a system comprising a biomedical device,
Figure 2C: a longitudinal cross section of a system comprising the third embodiment of the biomedical device according to Fig. 2A and the actuation unit according to Fig. 2B,
Figures 3A to 3F: longitudinal cross-sectional views of the third embodiment of the biomedical device according to Fig. 2A being introduced through a skin of a patient,
Figure 4: a longitudinal cross-sectional view of a fourth embodiment of the biomedical device comprising an insertion guide,
Figures 5A and 5B: schematic side views of a fifth embodiment of the biomedical device with an attachment mechanism,
Figure 5C: a cross-sectional view of a sixth embodiment of the biomedical device with a retaining structure,
Figures 5D to 5F: cross-sectional views of a first and a second embodiment of a retaining structure of the puncturing element according to Fig. 5C,
Figures 6A to 6D: longitudinal cross-sectional views of a first and second embodiment of a dilator connected to a puncturing element comprising an actuating element in different states,
Figure 7: a longitudinal cross-sectional view of an embodiment of the biomedical device comprising a mechanical coupling,
Figures 8A to 8C: longitudinal cross-sectional views of an embodiment of a puncturing element which has an anchoring member,
Figures 8D to 8F: longitudinal cross-sectional views of the embodiment of the puncturing element with the anchoring member according to Fig. 8A to 8C arranged within a dilator which is configured to convert the anchoring member,
Figures 8G and 8H: front views of a first and second embodiment of the puncturing element with the anchoring member according to Figs. 8A to 8C,
Figures 9A to 9C: a patient with a biomedical device being inserted into the femoral artery,
Figure 10: a first embodiment of a system comprising a biomedical device for insertion into the body duct of a patient which has imaging means and guidance means,
Figures 11A and 11B: a schematic side view of a first and second embodiment of the system comprising the biomedical device which has imaging means fixed to an introducer assembly and movable relative to an introducer assembly,
Figures 12A to 12C: a schematic side view of an introducer positioner with five, four, and three degrees of freedom for positioning the introducer assembly,
Figures 13A to 13D: a schematic side view of an embodiment of the imaging means relative to the body duct and method of positioning the imaging means relative to the body duct.

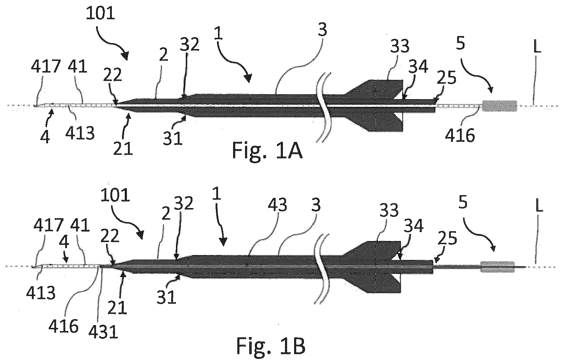

Figures 1A and 1B show longitudinal cross-sectional views of a first and second embodiment of a biomedical device 101 comprising an introducer assembly 1.

The introducer assembly 1 has an outer sheath 3, a dilator 2, and an inner guiding unit 4 arranged concentrically within each other along a longitudinal direction L of the introducer assembly 1. The sheath 3 circumferentially encloses the dilator 2 along its longitudinal length. It has a distal opening 32 at its distal end. The sheath 3 has a tapered section 31 at an outer surface neighboring the distal end and wings 33 for guiding the sheath 3 at a proximal end. The sheath 3 has a proximal opening 34 at its proximal end.

The dilator 2 extends through the distal opening 32 and the proximal opening 34 of the sheath 3.

The dilator 2 further has a distal opening 22 at its distal end. The distal end has a tapered outer section 21 for insertion through a skin and tissue into a body duct of a patient over the guiding unit 4. The dilator further has a proximal opening 25 at its proximal end. The dilator 2 circumferentially encloses the guiding unit 4 along its length.

The guiding unit 4 in Fig. 1A and 1B protrudes from the first and second opening 22, ,25 of the dilator 2. The sheath 3, the dilator 2, and the guiding unit 4 are all movable relative to each other in the longitudinal direction of the introducer assembly 1.

The guiding unit 4 is Fig. 1A comprises a puncturing element 41 formed by a continuous stainless-steel cannula which circumferentially encloses a cylindrical lumen 413 and has a puncturing section 417 at its distal end. The lumen 413 extends from a distal opening formed by the puncturing section 417 to a proximal end 416 at a proximal end 5 of the introducer assembly 1.

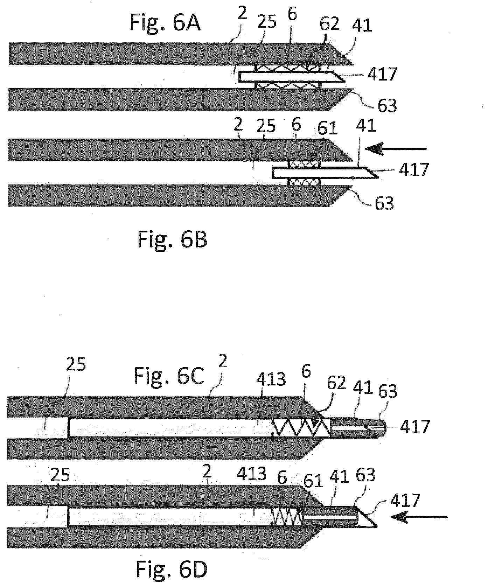

The cannula 41 can have a length in a range from 6 cm to 35 cm, in particular 15 cm. The cannula 41 in Fig. 1A is sufficiently stiff, such that the tapered outer section 21 of the dilator 2 can be introduced along the cannula 41 without requiring a separate guidewire. The cannula 41 can optionally be a Veress cannula (see Fig. 6B) for shielding the puncturing section 417 to protect a tissue wall of the body duct of the patient during introduction of the dilator 2 and/or the sheath 3.

The guiding unit 4 in Fig. 1B comprises a puncturing element formed by a continuous cylindrical stainless-steel cannula 41 and an additional guidewire 43. The cannula 41 has a shorter length than in Fig. 1A in a range from 0.1 cm to 6 cm, in particular 0.5 cm. The cannula 41 has a distal opening formed by the puncturing section 417 at its distal end and circumferentially encloses a cylindrical lumen 413. A proximal end 416 of the cannula 41 forms a smooth transition with a distal end 431 of the guidewire 43. The cannula 41 connected to the guidewire 43 can thus be moved back and forth relative to the remaining introducer assembly 1 by the guidewire 43 acting as a kind of push rod. The guidewire 43 has a lower bending stiffness than the cannula 41, so that it can more easily follow the turns in the introducer assembly 1 and/or the body duct. The guidewire 43 is sufficiently stiff, such that the tapered outer section 21 of the dilator 2 can be introduced into the body duct along the guidewire 43.

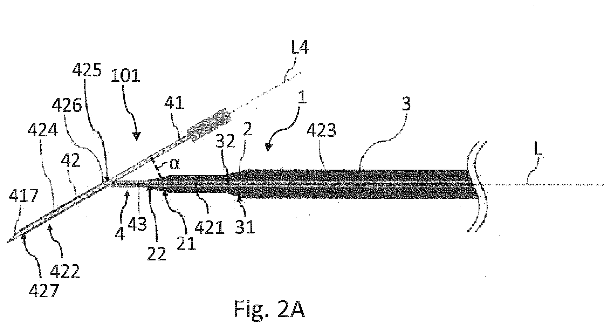

Figure 2A shows a longitudinal cross-sectional view of a third embodiment of a biomedical device 101 comprising,an introducer assembly 1 with an insertion guide 42. The introducer assembly 1 comprises a sheath 3, a dilator 2, and a guiding unit 4 which have sections which are arranged coaxially to each other along a longitudinal direction L of the introducer assembly 1. The sheath 3 has a distal opening 32 and a tapered outer section 31 on an outer surface near its distal end. The dilator 2 also has a distal opening 22 and a tapered outer section 21 on an outer surface near its distal end.

The insertion guide 42 of the guiding unit 4 has a proximal section 421 and a distal section 422. The proximal section 421 of the insertion guide 42 is circumferentially enclosed by the dilator 2 and has a proximal lumen 423 inside which a guidewire 43 is arranged. The distal section 422 of the insertion guide 42 has a distal lumen 424 which has a distal opening 427 at its distal end and a lateral opening 425 at its proximal end 426.

The distal lumen 424 extends in a straight line between the openings 425, 427. A puncturing element formed by a cannula 41 is arranged within the lumen 424 and is movable relative to the insertion guide 42 in the longitudinal direction L4 of the cannula 41.

The cannula can be a commercially available standardized stainless-steel cannula 41 which is not necessarily a part of the device. The distal section 422 is arranged at an angle α of 30° relative to the proximal section 421, such that the cannula 41 can be introduced and retracted from the lateral, opening 425 of the distal section 422 along the longitudinal direction L4 for introduction of the guidewire 43.

Therefore, the step of introducing the guidewire 43 can be carried out faster, since the cannula 41 only needs to be retracted from the lateral opening 425 over a short distance, such that the guidewire 43 can be introduced into the body duct. The cannula 41 is not arranged concentrically around the guidewire 43, such that the cannula 41 does not have to be removed from a proximal end of the introducer assembly 1 after the insertion of the cannula 41.

The insertion guide 42 can be introduced through a skin and tissue of a patient in parallel with the cannula 41 with a puncturing section 417 which protrudes from the distal opening 427 or be advanced around the cannula 41 was introduced into the body duct. The distal opening 427 of the insertion guide 42 instead forms a blunt edge such that the insertion.guide 42 is configured not to damage a tissue wall of the body duct.

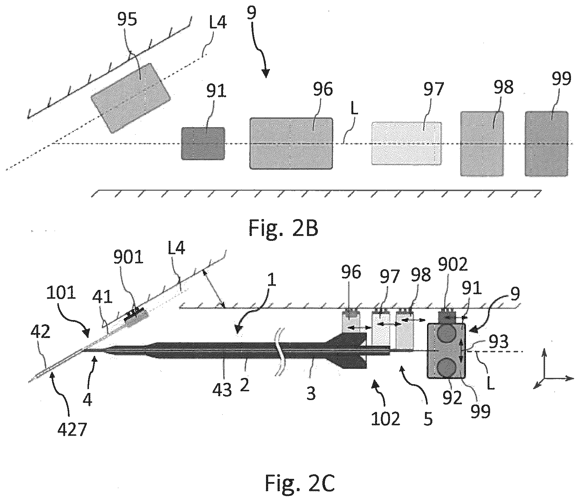

Figure 2B shows a schematic arrangement of an actuation unit 9 of a system comprising a biomedical device as described above. The actuation unit 9 has a clutch 91 which is switchable between different configurations 95, 96, 97, 98, 99 for grasping one of the components of the introducer assembly 1. In a first configuration 95 the actuation unit 9 or an additional actuation unit 9 is configured for grasping a cannula and moving the cannula relative to the remaining introducer assembly in a longitudinal direction L4.

In a second configuration 96, the actuation unit 9 is configured for grasping the sheath.

In a third configuration 97 the actuation unit 9 is configured for grasping the dilator.

In a fourth configuration 98 the actuation unit 9 is configured for grasping the insertion guide, and in a fifth configuration 99 for grasping the guidewire.

In one of these configurations 96, 97, 98, 99 the clutch 91 can move the respective grasped component of the introducer assembly in a longitudinal direction L relative to the remaining introducer assembly.

Alternatively to the actuation unit 9 of Fig. 2B at least some, in particular all, of the different components of the introducer assembly 1 may have an individual actuation unit which is configured for only actuating the according component in a longitudinal direction L, L4.

Figure 2C shows a longitudinal cross-sectional view of a system 102 comprising the third embodiment of the biomedical device 101 according to Fig. 2A and the actuation unit 9 according to Fig. 2B. The actuation unit 9 has a clutch 91 which comprises two rollers 92 which are adjustable to multiple different interdistances 93 and are arranged radially outward on two opposing sides of the introducer assembly 1.

The interdistance 93 between the rollers 92 in this embodiment is essentially arranged orthogonal to a longitudinal direction L of the insertion assembly 1. The sheath 3, dilator 2, and an insertion guide 42 and a guidewire 43 of the guiding unit 4 have at least partially exposed sections which are spaced from each other at a proximal end 5 of the insertion assembly 1. The actuation unit 9 comprises a longitudinal actuator 902 such that the clutch 91 can be moved in the longitudinal direction L of the introducer assembly 1, preferably along a rail parallel to the introducer assembly 1.

The longitudinal actuator 902 can be used to move the clutch to the partially exposed sections of the sheath 3, dilator 2, the insertion guide 42, and the guidewire 43. The interdistance 93 of the rollers 92 can then be adjusted to engage the partially exposed section. Thereby, a configuration 96, 97, 98, and 99 of the actuation unit 9 can be chosen to actuate the sheath 3, the dilator 2, the insertion guide 42, or the guidewire 43 in the longitudinal direction L by rotating the rollers 92 which frictionally engage the respective exposed section.

In Fig. 2C, a cannula 41 arranged within the distal section 427 of the insertion guide 42 is movable by a second actuation unit 901 along a longitudinal direction L4 at an angle relative to the longitudinal direction L of the introducer assembly 1.

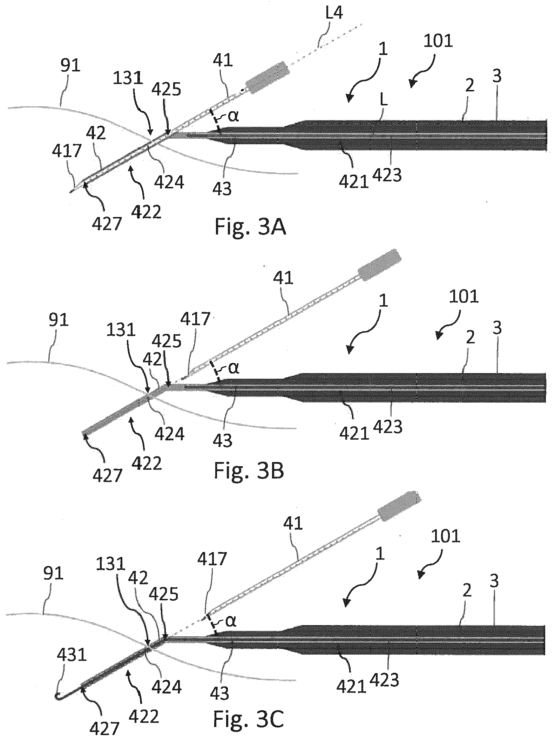

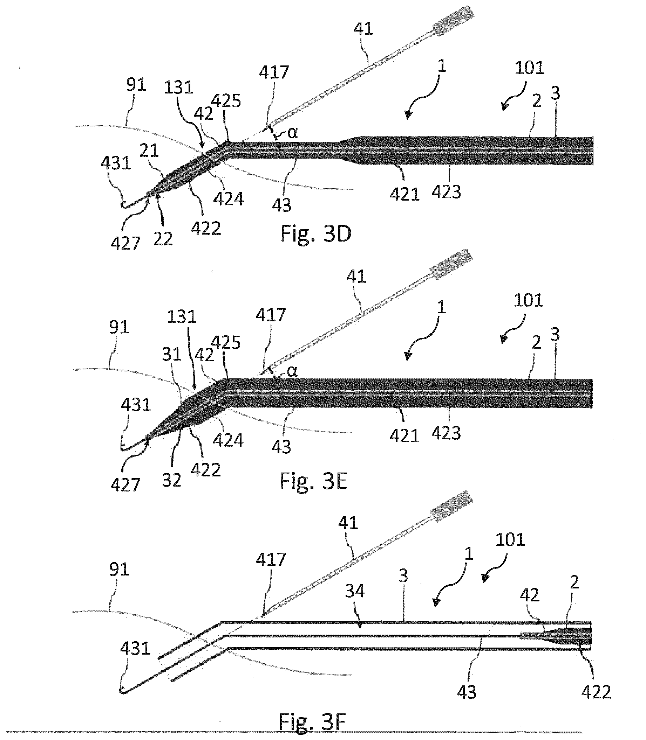

Figures 3A to 3F show longitudinal cross-sectional views of the third embodiment of the biomedical device 101 according to Fig. 2A introduced in several steps through a skin 91 of a patient at an insertion site 131.

In Fig. 3A, the cannula 41 is arranged within the distal lumen 424 of the distal section 422 of the insertion guide 42. The insertion guide 42 with the puncturing section 417 of the cannula 41 protruding from the distal opening 427 is introduced through the skin 91 in the longitudinal direction L4 of the cannula 41. The longitudinal direction L4 is arranged at the angle α of 30° relative to the longitudinal direction L (see Fig. 2A and Fig. 2C). The distal section 422 has a longitudinal length such that it is insertable into the body duct with the distal opening 427 and at the same time the lateral opening 425 of the distal section 422 is disposed outside the patient's skin 91. The longitudinal direction L4 of the distal section 422 can be arranged at a chosen angle with respect to a longitudinal axis of the body duct.

The guidewire 43 is arranged within the proximal section 423 of the insertion guide 42. The proximal section 423 is circumferentially surrounded by the dilator 2 and the sheath 3. An inner diameter of the dilator 2 corresponds to the outer diameter of the proximal section 421 and distal section 422 of the insertion guide 42.

Fig. 3B shows that the cannula 41 is retracted from the lateral opening 425 of the distal section 422, such that the distal lumen 424 is free from obstacles for inserting the guidewire 43.

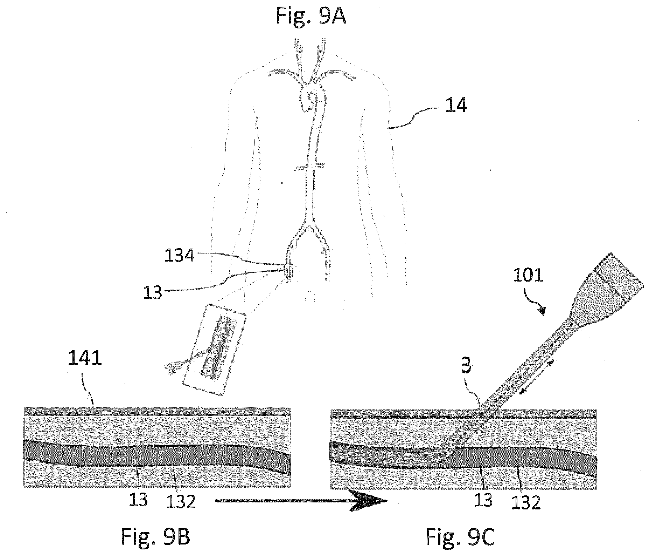

Fig. 3C shows that a distal end 431 of the guidewire 43 is introduced through the distal lumen 424 of the insertion guide 42 into the body duct (see Figs. 9B and 9C) of the patient.

Fig. 3D shows that the dilator 2 is introduced through the skin 91 of the patient with the tapered outer section 21 at its distal end. For this purpose, the distal opening 22 of the dilator is advanced over the insertion guide 42. The tapered outer section 21 of the dilator widens the insertion site 131, such that the dilator can be advanced.

Fig. 2E shows that the sheath 3 with the tapered outer section 31 is introduced through the skin of the patient with the distal opening 32 being advanced over the dilator 2 into the body duct.

Fig. 3F shows that the dilator 2 and the insertion guide 42 are retracted from a lumen 34 of the sheath 3, while the guide wire 43 stays in place, for performing a surgical procedure.

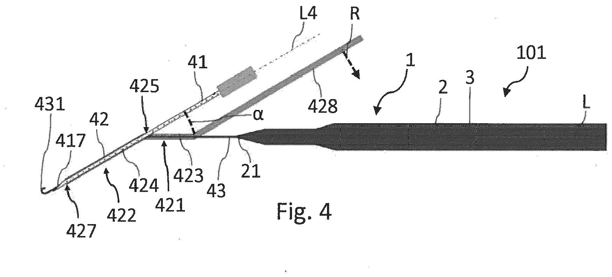

Figure 4 shows a longitudinal cross-sectional view of a fourth embodiment of a biomedical device 101 comprising and insertion assembly 1 which has an insertion guide 42. The device 101 is largely analogous to the device of Fig. 2A and reference is made to features which were previously described. However, in contrast to Fig. 2A the biomedical device 101 in Fig. 4 has a dilator 2 which is arranged coaxially enclosing a guidewire 43 instead of the insertion guide 42. An inner diameter of a lumen of the dilator 2 thus corresponds to the diameter of the guidewire 43. Therefore, a sheath 3, the dilator 2, and the guidewire 43 can be commercially available components or a set for performing the Seldinger technique. Apart from that the guidewire 43 can be introduced into a body duct through the insertion assembly 1 analogous to Figs. 3A to 3F.

The insertion guide 42 can be inserted using a puncturing surface 417 of a cannula 41 protruding from the distal opening 427 of the insertion guide 42. The cannula 41 can be advanced and retracted from the distal section 422 of the insertion guide 42 at an angle α relative to a longitudinal direction L of the introducer assembly 1. After retraction of the cannula 41 in the longitudinal direction L4 of the cannula 41, the guidewire 43 can be advanced into a body duct from the distal opening 427.

Fig. 4 shows a mantle section 428 of the insertion guide 42 being partially open in a circumferential area of the insertion guide 42, such that the guidewire 43 can be released from a proximal and a distal lumen 423, 424 of a proximal and the distal section 421, 422 in a radial direction R of the insertion guide 42. The partially open circumferential area extends over the entire length of the insertion guide 42.

Thereby, the insertion guide 42 can be separated from the guidewire 43 by retracting the insertion guide 42 relative to the guidewire 43, such that the guidewire 43 is radially released through the opening of the mantle surface 428. The insertion guide 42 is retracted along an additional translational path L3. Retracting the insertion guide 42 does not move the guidewire 43, such that a distal end 431 of the guidewire 43 can be retained in a body duct.

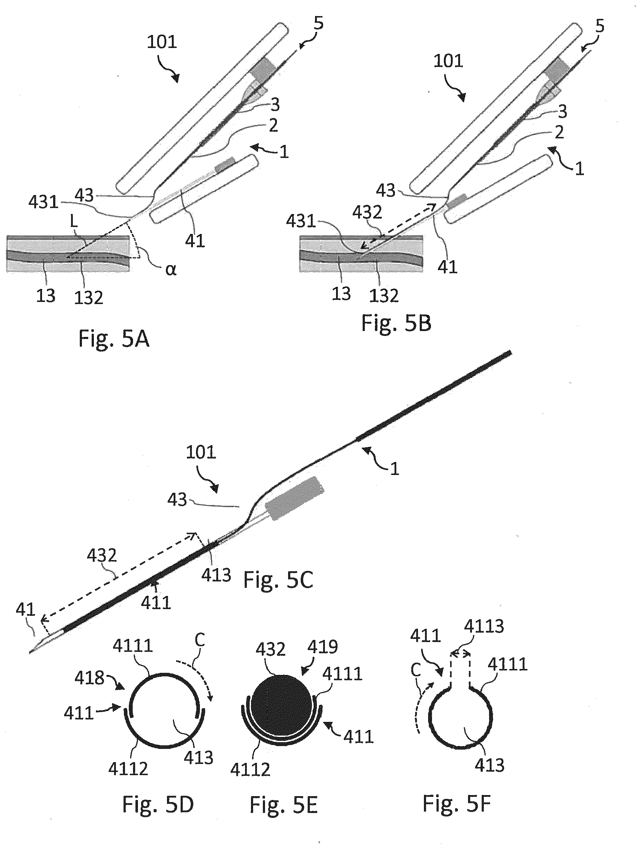

Figures 5A and 5B show schematic side views of a fifth embodiment of the biomedical device 101 comprising an introducer assembly 1 which has a guidewire 43 attached to a distal end 431 of a puncturing element 41. The distal end 431 comprises a spring-loaded attachment mechanism which attaches the guidewire 43 to the distal end 431, such that a longitudinal portion 432 of the guidewire 43 extends essentially parallel to a longitudinal direction L of the puncturing element 41. However, the guidewire 43 is arranged outside of a lumen of the puncturing element 41. The spring-loaded attachment mechanism is configured to retain the guidewire 43 during the insertion of the puncturing element 41 and to release the guidewire when passing through a tissue wall 132 of a body duct 13. Thereby, the guidewire 43 can be introduced into the body duct 13 without having to remove the puncturing element 41 at a proximal end 5 of the introducer assembly 1. A dilator 2 and a sheath 3 can be directly introduced into the body duct 13 subsequently or in parallel to the removal of the puncturing element 41.

Figure 5C shows a cross-sectional view of a sixth embodiment of the biomedical device 101 with an introducer assembly 1 which has a puncturing element 41 which has a retaining structure 411 (see Fig. 5D and Fig. 5E) that circumferentially encloses a guidewire 43 at least partially in a lumen 413 along a longitudinal portion 432 of the guidewire 43. The guidewire 43 in Fig. 5C is pre-loaded within the lumen 413.

Figures 5D and 5E show a first embodiment of the retaining structure 411 of the puncturing element 41 according to Fig. 5C. The retaining structure 411 in Fig. 5D has a first C-shaped mantle surface 4111 and a second C-shaped mantle surface 4112 which enclose the lumen 413 of the puncturing element 41 in a retaining state 418, such that the longitudinal portion 432 of the guidewire 43 (see Fig. 5C) can be retained in the lumen 413. The C-shaped mantle surfaces 4111, 4112 are rotatable relative with respect to each other in a circumferential direction C of the puncturing element 41 to convert the retaining structure 411 from the retaining state 418 to a radially open releasing state 419. The C-shaped mantle surfaces 4111, 4112 have different diameters such that the outer mantle surface 4112 in Fig. 4E encloses the inner mantle surface 4111. Thus, in a releasing state 419, the longitudinal portion 432 of the guidewire 43 can be released radially outwardly from the puncturing element 41.

Figure 5F shows a second embodiment of the retaining structure 411 of the puncturing element 41 according to Fig. 5C. The retaining structure 411 has a single mantle surface 4111 which only partially encloses the lumen 413 of the puncturing element 41 in a circumferential direction C of the puncturing element 41. The diameter of the guidewire can be larger or approximately the same as a circumferential size 4113 of a slot in the mantle surface (4111). The longitudinal portion of the guidewire 43 of Fig. 5C can be released radially outwardly from the lumen 413 by a applying a predetermined amount of force when retracting the puncturing element 41 relative to the guidewire.

Figures 6A to 6D show longitudinal cross-sectional views of a first and second embodiment of a dilator 2 connected to a puncturing element 41 comprising an actuating element 6. The actuating element 6 is formed by a spring which is convertible between a compressed state 61 in Fig. 6B and Fig. 6D and an extended state 62 in Fig. 6A and Fig. 6C.

Figures 6A and 6B show the puncturing element in the form of a cannula tip 41 being connected to the dilator 2 via the actuating element 6. The cannula tip 41 is arranged inside a lumen 25 formed by the dilator 2. In the expanded state 62 of the actuating element 6 a puncturing surface 417 of the cannula tip 41 is retracted behind a non-puncturing surface 63 formed by a distal end of the dilator 2. In the compressed state 61 of the actuating element 6 the puncturing surface 417 is advanced in a longitudinal direction of the dilator 2 such that the puncturing section 417 protrudes from the non-puncturing surface 63 of the dilator 2.

The figures 6C and 6D show the puncturing element in the form of a cannula tip 41 being directly connected to the dilator 2 within the inner lumen 25 of the dilator 2. The actuating element 6 is arranged within an inner lumen 413 of the cannula tip 41. In Fig. 6C the spring of the actuating element 6 is in an expanded state 61 such that a non-puncturing surface 63 located concentrically within the cannula is advanced forward to shield a puncturing section 417 of the cannula tip 41 analogously to a Veress cannula. Thereby, the cannula tip 41 cannot injure a wall of a body duct in the expanded state 61. In the retracted state 61 of the spring in Fig. 6D, the non-puncturing surface 63 located within the lumen 413 of the cannula tip 41 is retracted behind the puncturing surface 417, such that the cannula tip 41 can be introduced through a skin of a patient into the body duct.

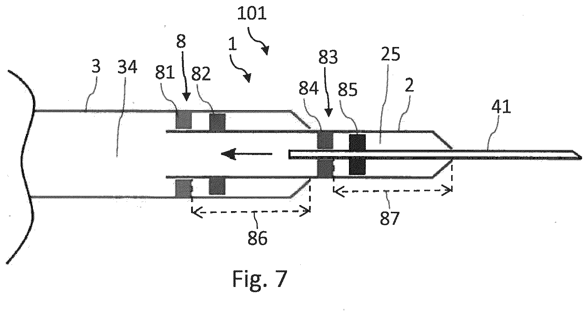

Figure 7 shows a longitudinal cross-sectional view of an embodiment of the biomedical device 101 comprising an introducer assembly 1 which has a sheath 3, a dilator 2, a cannula tip 41, and a first and a second mechanical coupling 8, 83. Such mechanical couplings 8, 83 can be arranged in any of the aforementioned first to fifth embodiments of the biomedical device 101.

The first coupling 8 has a first coupling element 81 formed by a radially inwardly projecting annular protrusion of the sheath 3 and a second coupling element 82 formed by a radially outwardly projecting annular protrusion of the dilator 2. Thereby, only a predetermined section 86 of the dilator 2 is insertable into a lumen 34 of the sheath 3 before the coupling elements 81, 82 block each other. The coupling elements 81, 82 can also be arranged the other way around such that only a predetermined section 86 of the dilator 2 can be introduced from the lumen 34.

The second coupling 83 has a first coupling element 84 formed by a radially inwardly projecting annular protrusion of the dilator 2 and a second coupling element 85 formed by a radially outwardly projecting annular protrusion of the cannula tip 41. Thereby, only a predetermined section 87 of the dilator 2 is insertable into a lumen 25 of the dilator 2 before the coupling elements 84, 85 block each other. The coupling elements 84, 85 can also be arranged the other way around such that only a predetermined section 87 of the puncturing element 41 can be introduced from the lumen 25.

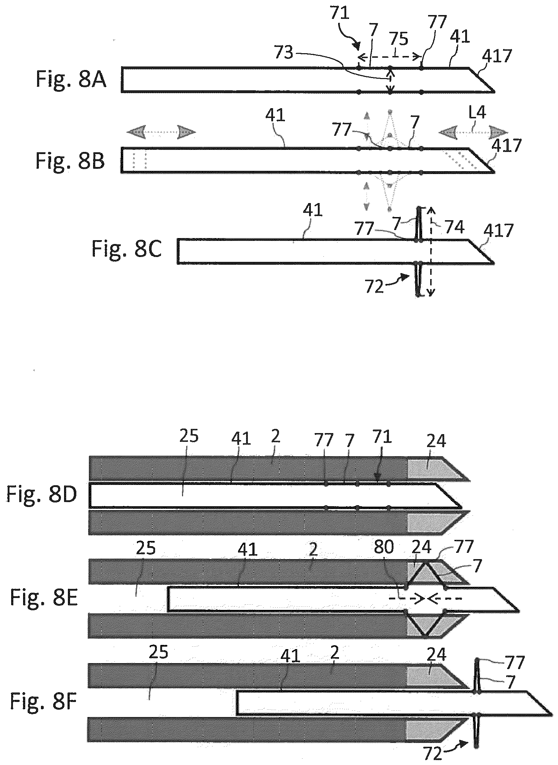

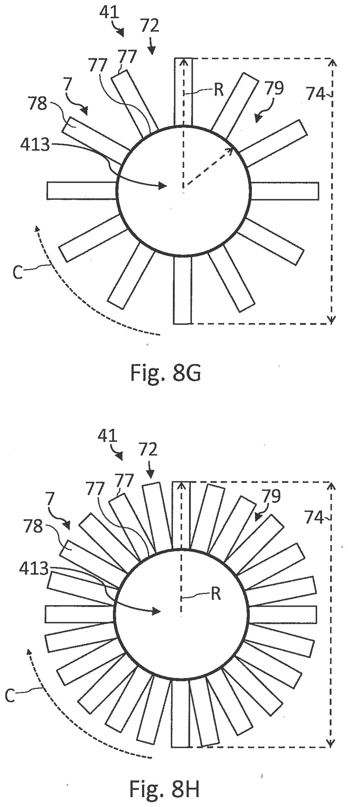

Figures 8A to 8C show longitudinal cross-sectional views of an embodiment of a puncturing element 41 which has an anchoring member 7. The anchoring member 7 is formed by a longitudinal section 75 of the puncturing element 41 and is arranged partially circumferentially around the puncturing element 41. Fig. 8A shows the anchoring member 7 in a collapsed configuration 71 which has a collapsed radial dimension 73. The anchoring member 7 is formed by three longitudinally spaced circumferential rows of joints 77 which allow the anchoring member 7 to be converted from the collapsed configuration 71 to an expanded configuration 72 (Fig. 8B). In the expanded configuration 72 in Fig. 8C, the anchoring member 7 forms a star-shape which has prongs formed by the center joints 77 located at the outermost radial tips of the anchoring member 7 (see Fig. 8G and Fig. 8H). Between the prongs of the anchoring member 7, the anchoring member 7 has longitudinal slots which essentially have a diameter of the collapsed radial dimension 73.

Fig. 8B further shows that the anchoring member 7 is biased to be converted from the collapsed configuration 71 to an expanded configuration 72. The anchoring member 7 is automatically converted by a restoring spring force of a spring element (not shown in Fig. 8A to Fig. 8F) of the anchoring member 7 in a longitudinal direction L4 of the puncturing element 41. The anchoring member 7 is only retained within the collapsed configuration 71 by a static retainment within the dilator 2 (see Fig. 8D).

The anchoring member 71 can be converted to the collapsed configuration 71 from the default expanded configuration 72 by advancing the dilator 2 over the anchoring member 7 (see Fig. 8D to 8F). The joints 77 of the anchoring member 7 allow for a radial expansion due to a longitudinal compression 80 based on the restoring spring force.

Fig. 8C shows the anchoring member 7 in the fully expanded configuration 72 having a maximum expanded radial dimension 74.

Figures 8D to 8F show longitudinal cross-sectional views of an embodiment of the puncturing element 41 with the anchoring member 7 according to Fig. 8A to Fig. 8C arranged within a lumen 25 of a dilator 2. The dilator 2 has longitudinal slots 24 at its distal end and is configured for converting the anchoring member 7 from a collapsed configuration 71 to an expanded configuration 72 and vice versa. The longitudinal slots 24 allow the dilator 2 to be advanced over prongs 78 of the anchoring member 7 to convert the anchoring member 7 from the default expanded configuration 72 to the collapsed configuration 71 (see Fig. 8G and Fig. 8H) .

The anchoring member 7 has an anchor actuator (not shown in Fig. 8D to 8F), such that the anchoring member 7 can be converted from the collapsed configuration 71 in Fig. 8D to the expanded configuration 72 in Fig. 8F by retracting the dilator 2 over the puncturing element 41 or advancing the puncturing element 41 relative to the dilator 2. The anchor actuator of the dilator 2 can engage the anchoring member 7, such that advancing the slots 24 over the anchoring member 7 can convert the anchoring member 72 from the expanded configuration 72 to the collapsed configuration 71 by applying a force directed outward in the longitudinal direction.

Figures 8G and 8H show front views of a first and a second embodiment of the puncturing element 41 with the anchoring member 7 of Figs. 8A to 8C in the expanded configuration 72. The anchoring member 7 in the expanded configuration 72 comprises prongs 78 which are formed by the longitudinal section 75 (see Fig. 8A) and have an expanded radial dimension 74. Each prong 78 is formed by joints 77 which are arranged on the distal tips of the prongs 77 and on the distal and proximal ends of the longitudinal section 75 (see Figs. 8A to 8C). Between neighboring prongs 78 the puncturing element 41 forms slits 79 in a longitudinal direction of the puncturing element 41, such that the longitudinal slots 24 of the dilator 2 (see Figs. 8D to 8F) can be advanced over the prongs 78. The slits 79 have a smaller width in a radial direction R of the puncturing element 41 than the width of the prongs 78 in the radial direction of the anchoring member 7. The slits 79 in Fig. 8G and 8H have essentially the same radial dimension as an inner lumen 413 of the puncturing element 41.

The prongs 77 of the anchoring means 7 can be arranged spaced apart from each other or directly adjacent to each other along a circumferential direction C of the puncturing element 41 as shown in Fig. 8G and in Fig. 8H.

Figure 9A shows a patient 14 and body ducts 13 of the patient 14 in which a biomedical device 101 is introducible. The section of a femoral artery 134 of the patient 14 into which a biomedical device 101 is inserted is shown in an enlarged view in Fig. 9B and Fig. 9C.

Figure 9B shows the body duct 13 of the femoral artery 134 enclosed by a tissue wall 132 and a skin 141 of the patient 14.

Figure 9C shows the sheath 3 of the device 101 introduced into the body duct 13, such that the catheterization is complete, and a subsequent medical procedure can be performed.

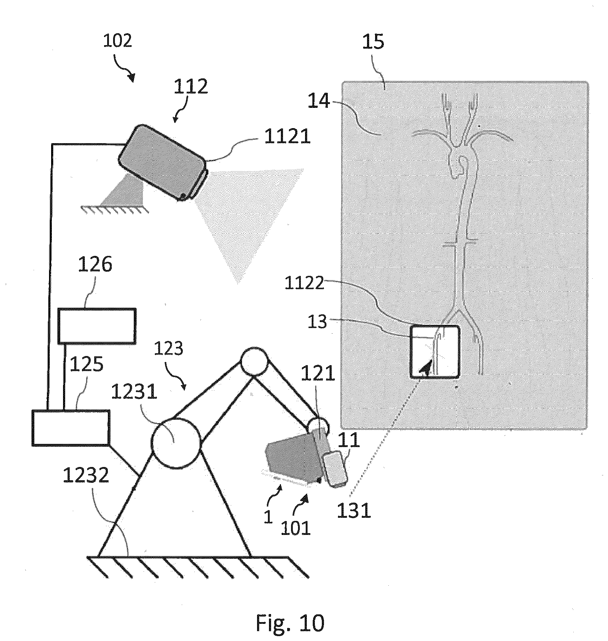

Figure 10 shows a schematic first embodiment of a system 102 comprising a biomedical device 101 for insertion into a body duct 13 of a patient 14. The biomedical device 101 has an ultra-sound probe 11 as an imaging means. The system 102 has a camera 1121 as guidance means 112. The device 101 comprises the ultra-sound probe 11 and the introducer assembly 1 connected via a base plate as a common support 121. The device 101 is arranged connected to a robotic arm 123 of the system 102.

The system 102 further has a processing unit 125 which is operatively connected to an actuator of the robotic arm 123, the imaging means 11, and the camera 1121. The processing unit 125 is configured to operate the robotic arm 123 based on the images provided by the ultrasound probe 11 and the camera 1121.

The processing unit 125 is further connected to a memory unit 126 which is configured to save images of the ultrasound probe 11 and the camera 1121 and processed data of the processing unit 125 based on these images. In addition, preoperative CT data of the body duct 13 are stored on the storage unit 126, such that the processing unit 125 can register the images provided by the ultrasound probe 11 to the CT data.

The robotic arm 123 is positioned on a stationary surface 1232 and has multiple joints 1231 such that the device 101 can be positioned relative to the body duct 13 at an insertion site 131.

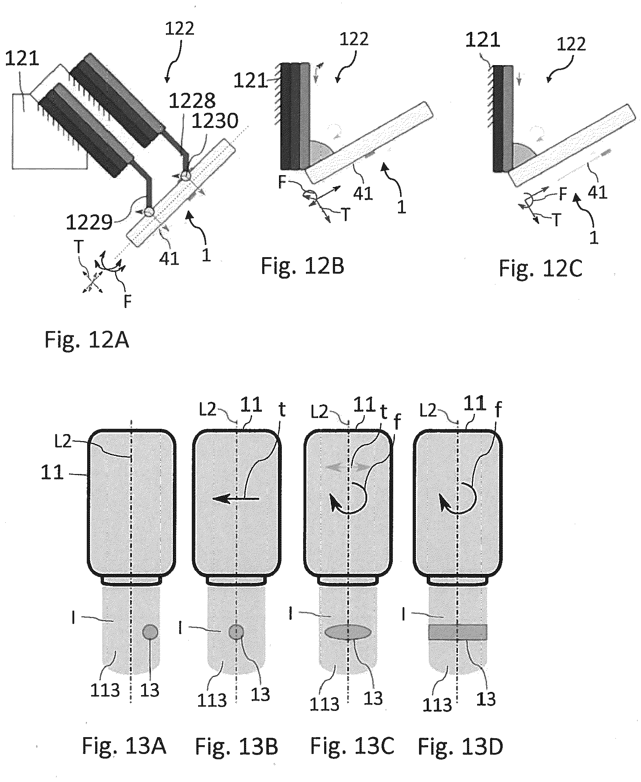

The camera 1121 supplies images to the processing unit 125. The processing unit 125 is configured to visually detect a landmark 1122 in the form of a known geometric shape, in particular a rectangular shape, within the supplied images. The landmark 1122 is placed on a drape 15 and frames the target site 131 for catheterization of the body duct 13, such that the target site 13 can be identified from the processing unit 125 based on the landmark 1122. The drape 15 covers the patient 4 except for the insertion site 131 to reduce unnecessary visual input for the camera 1121. Based on an input of the processing unit 125, the robotic arm 123 can position the device 101, such that the ultrasound probe 11 can be placed adjacent the body duct 13. For this purpose, the robotic arm 123 has three translational degrees of freedom t and two rotational degrees of freedom f (see Figs. 13A and 13B). Furthermore, the robotic arm 123 is configured for rotating the device 101 in a circumferential direction of the ultrasound probe 11 (see Figs. 13C to 13D).

The processing unit 125 is further configured to detect calcifications within the body duct 13. Based on the images provided to the processing unit 125 by the ultrasound probe 11 and a preoperative CT scan, the position of the device 101 can be adjusted using the robotic arm 123 to a different position if a predicted introduction path would pass through the detected calcifications.

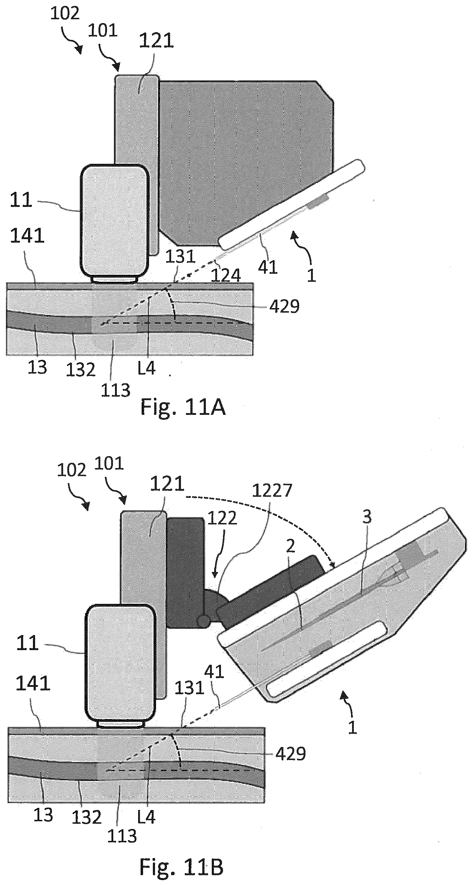

Figures 11A and 11B show schematic side views of a first and second embodiment of a system 102 comprising the biomedical device 101 which has an ultrasound probe 11 and an introducer assembly 1 fixedly connected to a baseplate 121. In Figs. 11A and 11B a longitudinal axis of the ultrasound probe 11 is positioned essentially orthogonal to a longitudinal axis of the body duct (see Figs. 13A to 13D). A field of view 113 of the ultrasound transducer is located at a section of the body duct 13 in which a puncturing element 41 of the introducer assembly 1 is introducible into the body duct 13 through an insertion site 131, a skin 141 of a patient, and a tissue wall 132 of the body duct 13.

In Fig. 11A, an angle α between a longitudinal direction L4 of the puncturing element 41 and the longitudinal direction of the body duct is fixed at 30°.

In Fig. 11B the angle α is adjustable by an electric motor 1227 of an introducer positioner 122 (see Figs. 12B to 12C). Positioning the introducer assembly 1 and in particular adjusting the longitudinal direction L4 of the puncturing element 41 does not move the transducer 11. A dilator 2 and a sheath 3 of the introducer assembly 1 can be introduced into the body duct 13 analogously as previously described in Fig. 1A.

Figures 12A to 12C show a schematic side view of an introducer positioner 122 with five (Fig. 12A), four (Fig. 12B), and three (Fig. 12C) degrees of freedom T, F for positioning the introducer assembly 1. The introducer positioner 122 is fixedly connected to a common support 121 which connects the introducer positioner 122 to the imaging means (see Figs. 11A and 11B).

Figure 12A shows an introducer positioner 122 with three translational degrees of freedom T and two rotational degrees of freedom F of the introducer assembly 1 relative to the common support 121. The arms 1228, 1229 each have a joint 1230 at an end connected to the introducer assembly 1, such that the introducer assembly 1 is rotatable relative to the common support 121. The introducer assembly 1 with a puncturing element 41 has the three translational degrees of freedom T and two rotational degrees of freedom F.

Figure 12B shows an introducer positioner 122 which has three translational degrees of freedom T and one rotational degree of freedom F.

Figure 12C shows an introducer position 122 which is essentially analogous to the introducer positioner 122 of Fig. 12B but the introducer assembly 1 in Fig. 12C only has two instead of three translational degrees of freedom T.

Figures 13A to 13D show schematic side views of an embodiment of an ultrasound probe 11 relative to a body duct 13 and a method of positioning the ultrasound probe 11 relative to the body duct 13. In Fig. 13A the ultrasound probe 11 is placed to image the body duct 13 by the device positioner 123 (see Fig. 10). The ultrasound probe 11 is preferably configured to retrieve three-dimensional spatial information with a minimum delay at more than 10 frames per second with a total data delay of less than 100 ms.

In a first step in Fig. 13A, a field of view 113 of the ultra-sound probe 11 is roughly aligned to the body duct 13, such that the field of view 113 captures a cross-section of the body duct 13 within an imaging plane I of the ultrasound probe 11.

In a second step in Fig. 13B, the ultrasound probe 11 is aligned, such that the cross-section of the body duct 13 is centered in the field of view 113 within the imaging plane I. Therefore, the ultrasound probe 11 is adjusted in at least one translational degree of freedom t by the device positioner, such that the imaging plane I is essentially orthogonal relative to a longitudinal direction of the body duct 13. Alternatively, the imaging plane I can be arranged such that the cross-section of the body duct 13 is at least fully contained within the field of view 113 of the ultrasound probe 11.

In a third step in Fig. 13C, the ultrasound probe 11 is adjusted in translational degrees of freedom t and rotational degree of freedom f until the longitudinal direction of the body duct 13 is essentially parallel to the imaging plane I as shown in Fig. 13D. In Fig. 13D the longitudinal direction of the body duct 13 is aligned essentially orthogonal to the longitudinal direction L2 of the ultrasound probe 11. During the alignment according to Fig. 13C, the translational and/or rotational degree of freedom f, t is continuously adjusted, such that the body duct 13 remains essentially centered in the field of view 113.

## Claims

1. Biomedical device (101) for percutaneous catheterization of a body duct (13), in particular arterial catheterization, the device (101) comprising an introducer assembly (1) which has
- a dilator (2) for widening of an insertion site (131) of a patient (14) to access the body duct (13),
- a sheath (3) for introduction into the body duct (13), and
- an inner guiding unit (4) for guiding at least one of the dilator (2) and the sheath (3) into the body duct (13),
the dilator (2) having a tapered outer section (21) and at least one distal opening (22), and being arranged at least partially concentrically around the guiding unit (4) and
the sheath (3) having at least one distal opening (32) and being arranged or arrangeable at least partially concentrically around the dilator (2), such that the guiding unit (4), the dilator (2), and the sheath (3) are movable relative to each other in a longitudinal direction (L) of the introducer assembly (1),
**characterized in that** once a puncturing element (41) is inserted into the body duct (13), the dilator (2) may be guided along the guiding unit (4) without complete withdrawal of the puncturing element (41) from a proximal end (5) of the introducer assembly (1).

2. Device (101) according to claim 1, wherein the guiding unit (4) comprises an for inserting at least one of the puncturing element (41) and a guidewire (43),
the insertion guide (42) having a and a distal section (422),
wherein the proximal section (421) has a proximal lumen (423) for at least partially receiving at least one of the puncturing element (41) and the guidewire (43) and
the distal section (422) has a distal lumen (424) for receiving the at least one of a puncturing element (41) and the guidewire (43),
the distal lumen (424) and the proximal lumen (423) being connected to each other,
the distal section (422) having
a lateral opening (425) at a proximal end (426) through which the puncturing element (41) can be reversibly inserted into the distal lumen (424) and
a distal opening (427) from which the puncturing element (41) can be inserted into the body duct (13) with the puncturing element (41).

3. Device (101) according to claim 2, wherein the distal lumen (424) extends in a straight line between the lateral opening (425) and the distal opening (427) of the distal section (422).

4. Device (101) according to one of the claims 2 or 3, wherein a longitudinal axis of the proximal section (421) of the insertion guide (42) is arranged or arrangeable at an angle of 1° to 125°, preferably an angle of 10° to 75°, more preferably 20° to 50°, relative to a longitudinal axis of the distal section (422) of the insertion guide (42).

5. Device (101) according to one of the claims 2 to 4, wherein the dilator (2) is one of
- arranged or arrangeable coaxially around the insertion guide (42) and configured to be advanced relative to the insertion guide (42) in a longitudinal direction of the insertion guide (42) and
- arranged or arrangeable coaxially around the guidewire (43) and configured to be advanced relative to the guidewire (43) in a longitudinal direction of the guidewire (43), wherein a mantle section (428) of the proximal and distal section (421, 422) has a release opening extending in a longitudinal direction, the release opening being permanently open or openable and configured for radially releasing the guidewire (43) which is at least partially arranged or arrangeable in the proximal and distal lumen (423, 424).

6. Device (101) according to one of the preceding claims, wherein the guiding unit (4) comprises or consists of a puncturing element (41), and optionally the insertion guide (42) or guidewire (43).

7. Device (101) according to claim 6, wherein the puncturing element (41) comprises at least one retaining structure (411) which has at least one of:
- a recess (412) arranged on an outer surface and being open radially outwardly (R) (41) for receiving a guidewire (43) extending at least partially along a longitudinal direction (L4) of the puncturing element (41),
- a lumen (413) defined by a wall which is at least partially open or openable for radially releasing a guidewire (43) which is at least partially arranged or arrangeable in the lumen (413), and
- an attachment mechanism for attaching a guidewire (43) in parallel to the puncturing element (41).

8. Device (101) according to one of the claims 6 or 7, wherein the puncturing element (41) is relatively long and has
- a longitudinal length (415) of 6 cm to 35 cm, preferably 8 cm to 30 cm, particularly 12 cm to 25 cm, and/or
- a connection of a proximal end (416) to a distal end (431) of a guidewire (43) such that the puncturing element (41) and the guidewire (43) are essentially flush with each other.

9. Device (101) according to one of the claims 6 to 8, wherein the puncturing element (41) is connected or connectable to the dilator (2) and comprises an actuating element (6), in particular a spring, which is convertible between a retracted state (61) and an extended state (62), wherein by conversion of the actuating element (6), a puncturing section (417) of the puncturing element (41) for contacting and puncturing tissue is retractable behind a non-puncturing surface (63), or a non-puncturing surface (63) can be advanced for shielding the puncturing section (417).

10. Device (101) according to one of the claims 6 to 9, wherein the puncturing element (41) comprises an anchoring member (7) arranged at least partially circumferentially on the puncturing element (41) and preferably forming a longitudinal section (75) of the puncturing element (41),
the anchoring member (7) comprising a collapsed configuration (71) and an expanded configuration (72), in the collapsed configuration (71), the anchoring member (7) has a radial dimension (73) which is smaller than a radial dimension (74) in the expanded configuration (72), such that
the anchoring member (7) in the expanded configuration (72) prevents advancement of the puncturing element (41) into the patient (14), in particular into the body duct (13), beyond a predetermined longitudinal distance of the puncturing element (41) and/or prevents retraction of the puncturing element (41) from the patient (14), in particular from the body duct (13), beyond a predetermined longitudinal distance of the puncturing element (41).

11. Device (101) according to claim 10, wherein the dilator (2) comprises an anchor actuator, such that upon a longitudinal movement of the dilator (2) relative to the puncturing element (41), the anchor actuator engages the anchoring member (7) in the expanded configuration (72) to convert the anchoring member (7) from the expanded configuration (72) to the collapsed configuration (71).

12. Device (101) according to claim 11, wherein the dilator (2) comprises longitudinal slots (24) arranged at the distal opening (22) of the dilator (2), wherein the longitudinal slots (24) are configured to be advanced past the anchoring member (7) in the longitudinal direction (L4) of the puncturing element (41).

13. Device (101) according to one of the preceding claims,
wherein at least two of, optionally three of, the sheath (3), the dilator (2), and at least a part of the guiding unit (4), in particular the puncturing element (41), are connected to at least one mechanical coupling (8),
such that a relative longitudinal movement of at least one of the sheath (3), the dilator (2), or at least the part of the guiding unit (4) beyond a predetermined point relative to the others of the sheath (3), the dilator (2), and/or the part of the guiding unit (4), causes the coupling (8) to move at least one other of the sheath (3), the dilator (2), or the part of the guiding unit (4) in the longitudinal direction (L) of the introducer assembly (1).

14. A system (102) comprising a biomedical device (101), preferably a biomedical device (101) according to one of the preceding claims, for percutaneous catheterization of a body duct (13), in particular arterial catheterization, wherein the device (101) has an introducer assembly (1) having
- a dilator (2) for widening of an insertion site (131) of a patient (14) to access the body duct (13),
- a sheath (3) for introduction into the body duct (13), and
- an inner guiding unit (4) for guiding at least one of the dilator (2) and the sheath (3),
the dilator (2) having a tapered outer section (21), a distal opening (22), and being arranged at least partially concentrically around the guiding unit (4) and
the sheath (3) having a distal opening (32) and being arranged at least partially concentrically around the dilator (2), such that the guiding unit (4), the dilator (2), and the sheath (3) are movable relative to each other in a longitudinal direction (L) of the introducer assembly (1),
wherein the system (102) comprises at least one actuation unit (9) which is configured to actuate at least one of, in particular two of, preferably all of the dilator (2), the sheath (3), the guiding unit (4), a puncturing element (41), a guidewire (43) and an insertion guide (42), of the device (101) in a longitudinal direction of the introducer assembly (1).

15. System (102) according to claim 14, wherein the system (102) comprises one actuation unit (9), preferably exactly one actuation unit (9), which comprises a clutch (91), in particular a clutch (91) comprising at least two rollers (92) which have an adjustable interdistance (93),
such that the actuation of the actuation unit (9) can be switched between configurations (95, 96, 97, 98, 99) for actuating at least two, preferably three, in particular preferably all of, the dilator (2), the sheath (3) and the guiding unit (4), in particular the puncturing element (41), the guidewire (43), and/or an insertion guide (42).

16. System (103) comprising a biomedical device (101), in particular a biomedical device (101) according to one of the claims 1 to 13, for percutaneous catheterization of a body duct (13), in particular arterial catheterization,
wherein the device (101) has a positionable introducer assembly (1) for puncturing an application site at a patient's skin, and an external imaging means (11), in particular one of
- an ultrasound probe, in particular a Doppler ultrasound probe (111) for measuring a blood flow, and
- a X-ray imaging probe,
for imaging a body duct (13) of a patient (14), in particular an arterial body duct (13), in two dimensions or three dimensions, optionally in a time periodic measurement, wherein
the external imaging means (11) and the introducer assembly (1) are connected to a common support (121) of the system (103) .

17. System (103) according to claim 16, wherein the system (103) comprises an external guidance means (112), wherein the guidance means (112) have at least one of
- a stereo camera (1121) or a RGBD camera,
- a LIDAR system, and
- an electromagnetic sensor,
such that a distance of the patient (14), in particular the body duct (13), and/or a surrounding of the patient (14) to the imaging means (11) and/or the introducer assembly (1), in particular the guiding unit (4), can be determined or reconstructed.

18. System (103) according to claim 17, wherein the guidance means (112) are one of:
- immovably connected to the external imaging means (11),
- immovably connected to the patient (14) or a stationary surface (1123),
- attached to a movable surface (1124) which is independently movable from the imaging means (11).

19. System (103) according to one of the claims 16 to 18,
wherein the device (101) comprises an introducer positioner (122) for positioning of the introducer assembly (1) relative to the body duct (13) and/or the common support (121),
wherein the introducer positioner (122) is configured to position the introducer assembly (1) in one of:
- two translational degrees of freedom (t) and at least one rotational degree of freedom (f),
- three translational degrees of freedom (t) and at least one rotational degree of freedom (f), in particular two or three rotational degrees of freedom (f).

20. System according to one of the claims 16 to 19, wherein the common support (121) is
- A hand-held support (121) for manual alignment of the device (101), in particular the imaging means (11), relative to the patient (14),
- A spatially fixed support (121) which comprises a prepositioning means, in particular an adhesive, a strap, or a mechanically fixed or fixable carrier or
- A spatially movable support (121) which comprises a device positioner (123) for positioning of the device (101) relative to the body duct (13) independently in two or three translational degrees of freedom (t) and at least one rotational degree of freedom (f), in particular one, two, or three rotational degrees of freedom (f).

21. System (103) according to one of the claims 16 to 20, wherein the system (103) comprises an force sensor (124) which is configured for converting an external force, in particular an external torque, exerted on at least a subsection of the system (103), in particular a distal tip of the guiding unit (4), preferably a distal end (431) of the puncturing element (41) of the guiding unit (4), to an electrical signal indicative of the external force.

22. System (103) according to one of the claims 16 to 21, wherein the system (103) comprises a processing unit (125) for processing data relating to at least one of:
- the measured image of the body duct (13), in particular a plurality of images, of the external imaging means (11),
- the determined or reconstructed distance, in particular a plurality of distances, of the guidance means (112), and
- the electrical signal, in particular a plurality of electrical signals, of the force sensor (124),
to calculate, based on the data, at least one electrical signal for
- operating at least one of the introducer positioner (122) and the device positioner (123).

23. System (103) according to claim 22, wherein the system comprises a memory unit (126) connected or connectable to the processing unit (125) for saving data relating to at least one of
- the measured image of the body duct (13), in particular a plurality of images, of the external imaging means (11),
- the determined or reconstructed distance, in particular a plurality of distances, of the guidance means (112), and
- the electrical signal, in particular a plurality of electrical signals, of the force sensor (124).

24. Method for percutaneous'catheterization of a body duct (13) using a biomedical device (101) which has an introducer assembly (1), preferably according to one of the claims 1 to 13, comprising the following steps:
- Inserting an inner guiding unit (4) through a skin of a patient (14) into the body duct (13) using a puncturing element (41),
- Optionally inserting a guidewire (43) of the inner guiding unit (4) through the insertion guide (42) into the body duct (13),
- Guiding a dilator (2) along the inner guiding unit (4) without full removal of the puncturing element (41) at a proximal end (5) of the introducer assembly (1),
- Advancing a sheath (3) over the dilator (2) into the body duct (13),
- Optionally retracting at least one of the dilator (2), the inner guiding unit (4), and the guidewire (43).

25. Method for aligning the external imaging means (11) relative to a body duct (13), preferably using a system (103) according to one of the claims 16 to 23, comprising the steps of:
- imaging a plane (I) of the body duct (13) using the external imaging means (11),
- aligning the external imaging means (11) in translational degrees of freedom (t) and/or rotational degrees of freedom (f) of the imaging means (11), preferably using the device positioner (123), such that the body duct (13) is centred in a field of view (113) of the imaging means (11) and positioned along an orthogonal direction of the imaging plane (I) or in a range of 30°, in particular 15°, relative to the orthogonal direction,
- aligning the external imaging means (11) in the rotational and/or translational degrees of freedom (t), preferably using the device positioner (123), such that the body duct (13) remains centred in the field of view (113) and the imaging plane (I) is positioned at a parallel direction of a longitudinal axis of the body duct (13).
